# EUROPEAN PATENT APPLICATION

(11) **EP 2 842 597 A1**
(43) Date of publication of application: **04.03.2015**
(21) Application number: 13182382.5
(22) Date of filing: 30.08.2013
(51) Int. Cl.: A61N 1/04

(54) **Body electrode**

(71) Applicant: Mölnlycke Health Care AB, 402 52 Göteborg (SE)
(72) Inventor: Båtelsson, Erik, 426 76 Västra Frölunda (SE); Jacobsen, Carolin, 417 01 Göteborg (SE); Flach, Niclas, 441 35 Alingsås (SE); Roy, Pia, 434 36 Kungsbacka (SE); Bergstrand, Sofia, 434 46 Kungsbacka (SE); Wötzer, Stephan, 6170 Zirl (AT); Naier, Benjamin, 6700 Bludenz (AT); Wilfinger, Markus, 6063 Rum (AT)
(74) Representative: Valea AB

(57) **Abstract**

The present disclosure relates to a body electrode (14) comprising a body-facing layer (22), a conductive layer (24) and a conductive body (26). The conductive layer (24) comprises a first material composition and the conductive body (26) comprises a second material composition. The second material composition has a lower electrical resistance than the first material composition. The conductive body (26) is located within and/or on the conductive layer (24). The body electrode (14) comprises a plurality of fluid transport passages (30) extending through both of the body-facing layer (22) and the conductive layer (24).

## Description

### TECHNICAL FIELD

The present disclosure relates to a body electrode. Moreover, the present disclosure relates to a wound care treatment kit comprising a body electrode. Furthermore, the present disclosure relates to a method for treating a wound.

### BACKGROUND

Some types of wounds are advantageously treated by a treatment method in which electrical current is directed towards the wound. Such a treatment method may involve the attachment of a body electrode to the wound area.

The body electrode may have a body-facing layer that is adapted to be attached to the wound area. Moreover, the body-facing layer may be adapted to absorb exudate from the wound during the treatment process. However, there is a risk that the body-facing layer of the above discussed body electrode may be saturated with exudate and this may in turn result in that the body electrode may have to be replaced in order to continue the treatment.

In order to reduce the need for a body electrode replacement, US 5,974,344 proposes that openings be provided in the body-facing layer as well as in a conductive body that is attached to the body-facing layer. As such, during therapy, exudate may be transported from the wound area towards an absorbent article via the openings.

However, there is a risk that the '344 body electrode may provide an uneven distribution of the electrical current conducted to the body electrode.

### SUMMARY

An object of the present disclosure is to provide a body electrode that has an appropriate exudate transporting capability and that also may provide an appropriate distribution of electrical current to a wound area to be treated by the body electrode.

The above object is obtained by a body electrode according to claim 1.

As such, the present disclosure relates to a body electrode comprising a body-facing layer, a conductive layer and a conductive body. The conductive layer comprises a first material composition and the conductive body comprises a second material composition. The second material composition has a lower electrical resistance than the first material composition. The conductive body is located within and/or on the conductive layer. The body electrode comprises a plurality of fluid transport passages extending through both of the body-facing layer and the conductive layer.

By virtue of the conductive layer of the body electrode as presented hereinabove, it is possible to obtain an appropriate electrical current distribution to the wound area. For instance, the presence of a conductive layer may imply that an appropriately even electrical current distribution may be obtained.

Moreover, the feature that the body electrode comprises a conductive body that is located within and/or on the conductive layer implies that an appropriate conduction of electrical current to the conductive layer may be obtained. This in turn also implies that the electrical current can be conducted to various portions of the conductive layer which increases the possibilities to obtain an appropriate distribution of the electrical current in the conductive layer.

Optionally, the conductive body is attached to a first side of the conductive layer. The first side faces away from the body-facing layer.

Optionally, the conductive body is isolated from the environment ambient of the body electrode.

By virtue of the fact that the conductive body is isolated from the ambient environment, the risk that portions of the conductive body will dissolve, for instance due to contact with the exudate, and enter the wound area is reduced. As an example, if the conductive body comprises metal, there could be a risk that a portion of the metal could be ionized when in contact with the exudate, or any other liquid, and metal ions could then enter the wound area. However, such a risk is reduced when the conductive body is isolated from the environment ambient of the conductive body.

Optionally, the body electrode has a planar extension in a first plane extension and comprises, along the planar extension, a first body electrode portion and a second body electrode portion, the conductive body being located within the first body electrode portion.

Optionally, each one of the first and second body electrode portions comprises a portion of each one of the body-facing layer and the conductive layer. A configuration according to the above, wherein each one of the first and second body electrode portions comprises a portion of each one of the body-facing layer and the conductive layer, implies that the body electrode may be manufactured in a straightforward manner.

Optionally, the body-facing layer comprises a hydrogel.

Optionally, the hydrogel has a liquid content within the range of 25% to 50%.

Optionally, the body-facing layer has a thickness within the range of 0.1 to 1.0 mm. Alternatively, the body-facing layer has a thickness within the range of 0.1 to 0.4 mm

Optionally, the cross-sectional area of each one of the fluid transport passages is within the range of 0.5 to 30 mm².

Optionally, the first material composition comprises an intrinsic conductive carbon material. The intrinsic conductive carbon material may be formed as a foil in order to form at least a part of the body-facing layer. As an example, the first material composition may comprise a carbon polymer.

Optionally, the first material composition comprises an inert metal. The inert metal may be formed as a foil in order to form at least a part of the body-facing layer.

Optionally, the second material composition comprises a metal or an alloy.

Optionally, the second material composition is constituted by a metal or an alloy.

Optionally, the second material composition comprises a well conductive compound, for instance at least one of nanotubes, such as carbon nanotubes, fullerens or buckypapers. Optionally, the conductive body is attached to the first side of the conductive layer by means of screen printing and/or roll coating.

Optionally, the conductive body is isolated from the environment ambient of the body electrode by means of an isolating layer covering the conductive body.

Optionally, the conductive body, in the planar extension, forms a closed path.

Optionally, a minimum distance, along the planar extension, between the conductive body and each one of the fluid transport passages is within the range of 1 to 4 mm, alternatively within the range of 2 to 3 mm.

Optionally, the body electrode further comprises a lead adapted to conduct an electrical current from a source of electrical current to the conductive body.

Optionally, the lead comprises the second material composition.

A second aspect of the present disclosure relates to a wound care treatment kit. The kit comprises a body electrode according to the first aspect of the present disclosure. The kit further comprises an absorbent article adapted to be attached to the body electrode. At least a portion of the absorbent article is adapted to face a first side of the conductive layer. The first side faces away from the body-facing layer.

A third aspect of the present disclosure relates to a method for treating a wound in a wound area, the method comprising:
- applying a body electrode according to the first aspect of the present disclosure to the wound area such that the body-facing layer faces the wound;
- covering the body electrode with an absorbent article such that at least a portion of the absorbent article faces a first side of the conductive layer, the first side facing away from the body-facing layer, and
- applying an electrical current to the conductive body.

Optionally, the body electrode is applied to the wound area before the body electrode is covered with the absorbent article.

Optionally, the body electrode is covered with the absorbent article before the body electrode is applied to the wound area.

Optionally, the body electrode comprises a release liner initially covering at least the body-facing layer, the method comprising removing the release liner prior to applying the body electrode to the wound area.

Optionally, the method comprises fixating the body electrode and the absorbent article by means of a fixator.

Optionally, the method comprises connecting the lead to a source of electric current.

Optionally, the method comprises placing a disperser electrode on the body hosting the wound area at a disperser electrode distance from the wound area. The disperser electrode distance is greater than or equal to a predetermined disperser electrode distance value. Moreover, the method may comprise applying an electrical current to the disperser electrode.

### BRIEF DESCRIPTION OF THE DRAWINGS

With reference to the appended drawings, below follows a more detailed description of embodiments of the invention cited as examples.

In the drawings:
- Fig. 1: illustrates an embodiment of a wound care treatment kit;
- Fig. 2: illustrates an embodiment of a body electrode;
- Fig. 3: illustrates a cross-sectional view of the Fig. 2 electrode along line III - III of Fig. 2;
- Fig. 4: illustrates a cross-sectional view of another embodiment of a body electrode;
- Fig. 5: illustrates another embodiment of a body electrode;
- Fig. 6: illustrates a further embodiment of a body electrode;
- Fig. 7: illustrates another embodiment of a body electrode;
- Figs. 8 - 14: illustrate embodiments of a method for treating a wound using a body electrode.

It should be noted that the appended drawings are not necessarily drawn to scale and that the dimensions of some features of the present invention may have been exaggerated for the sake of clarity.

### DETAILED DESCRIPTION OF PREFERRED EMBODIMENTS

The invention will, in the following, be exemplified by embodiments. It is to be understood, however, that the embodiments are included in order to explain principles of the invention and not to limit the scope of the invention defined by the appended claims.

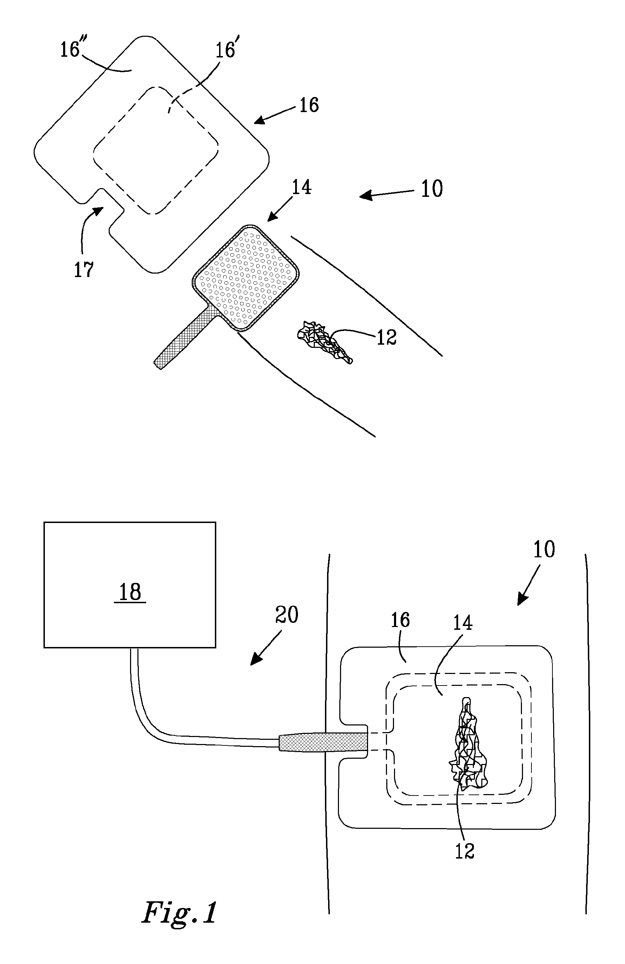

Fig. 1 illustrates an exploded perspective view of a wound care treatment kit 10 for treating a wound 12. The Fig. 1 wound care treatment kit comprises a body electrode 14 and an absorbent article 16. The body electrode 14 is adapted to be connected to a source of electrical current 18, for instance via a body electrode cable 20.

As may be gleaned from Fig. 1, though purely by way of example, the absorbent article 16 may comprise an absorbent pad 16', adapted to at least partially overlap the body electrode 14, and a backing layer 16". Purely by way of example, the backing layer 16" may comprise an adhesive such that the backing layer can be attached to an area around the wound 12 and/or to a portion of the body electrode 14. Moreover, as a non-limiting example, the absorbent article 16, for instance the backing layer 16" thereof, may comprise a notch 17 through which a portion of the body electrode 14 may extend.

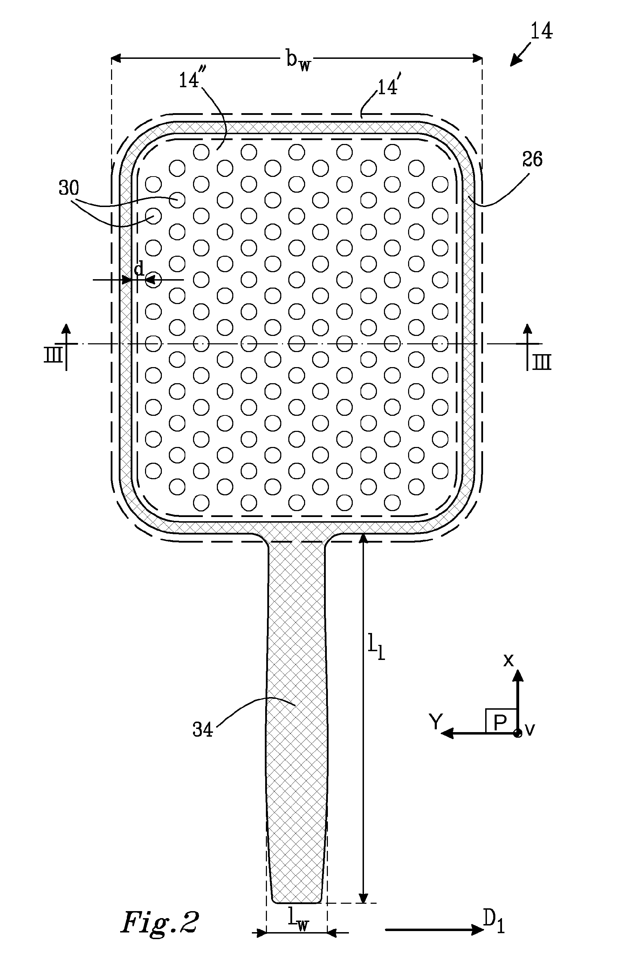

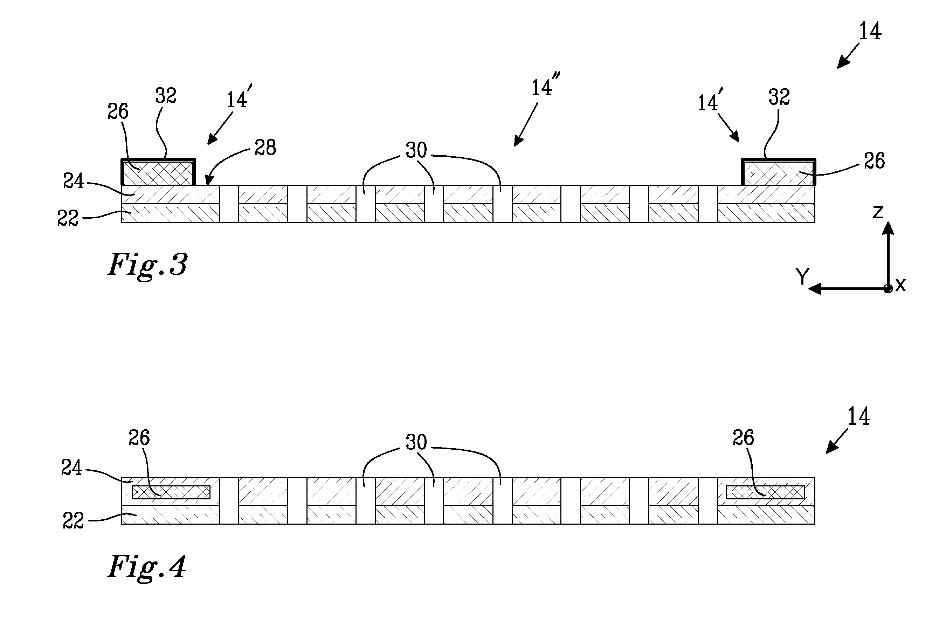

Fig. 2 and Fig. 3 illustrate an embodiment of a body electrode 14. As may be gleaned from Fig. 3, the body electrode comprises a body-facing layer 22, a conductive layer 24 and a conductive body 26.

Purely by way of example, the body-facing layer 22 may comprise a hydrogel. As a nonlimiting example, the hydrogel may have a liquid content within the range of 25% to 50%. In example embodiments of the body electrode 14, the hydrogel may have a liquid content that is approximately 30%. The liquid of the hydrogel may, as a non-limiting example, be water. The body-facing layer 22 may be adapted to adhere the body electrode 14 to the area that surrounds the wound 12.

Instead of, or in addition to a hydrogel, the body-facing layer 22 may comprise one or more other types of a conductive adhesives. Purely by way of example, the the body-facing layer 22 may comprise a conductive adhesive that has been produced by way of microemulsion. An example of such an adhesive is disclosed in US 2002/0188035 A1.

Moreover, though purely by way of example, the body-facing layer 22 may have a thickness within the range of 0.1 to 1.0 mm, alternatively within the range of 0.1 to 0.4 mm. In example embodiments of the body electrode 14, the hydrogel may have a thickness that is approximately 0.2 mm. A thickness within the above range may imply that the body-facing layer 22 adheres to at least a portion of the wound area in an appropriate manner and preferably also enables appropriate electrical contact between the wound area and the e.g. the conductive layer 24.

The conductive layer 24 comprises a first material composition. As a non-limiting example, the conductive layer may be constituted by the first material composition.

Moreover, the conductive body 26 comprises a second material composition. As a non-limiting example, the conductive body may be constituted by the second material composition.

The second material composition has a lower electrical resistance than the first material composition. In other words, the second material composition generally has a higher electrical conductivity than the first material composition.

Purely by way of example, the first material composition comprises an intrinsic conductive carbon material. The intrinsic conductive carbon material may be formed as a foil in order to form at least a part of the body-facing layer. Purely by way of example, the first material composition may comprise a carbon polymer. Instead of, or in addition to, the intrinsic conductive carbon material, the first material composition may comprise an inert metal. The inert metal may be formed as a foil in order to form at least a part of the body-facing layer.

Moreover, though purely by way of example, the second material composition may comprise a metal or an alloy. In embodiments of the body electrode, the second material composition is constituted by a metal or an alloy. Instead of, or in addition to the metal or alloy, second material composition may comprise a well conductive compound. Purely by way of example, such a well conductive compound may comprise at least one of nanotubes, such as carbon nanotubes, fullerens or buckypapers.

As non-limiting example, the second material composition may comprise one or more of the following metals: silver, copper, aluminium or titanium.

The conductive body 26 is located within and/or on the conductive layer 24. In the embodiment illustrated in Fig. 2 and Fig. 3, the conductive body 26 is located on the conductive layer 24. In fact, and as is intimated in Fig. 3, in the Fig. 2 and Fig. 3 embodiment, the conductive body 26 is attached to a first side 28 of the conductive layer 24, which first side 28 faces away from the body-facing layer 22.

The conductive body 26 may be attached to the conductive layer 24 in a plurality of ways. Purely by way of example, the conductive body 26 may be welded or glued to the conductive layer 24. As another alternative, the conductive body 26 may be screen printed and/or roll coated onto the conductive layer 24. Moreover, the conductive body 26 may comprise one or more wires, for instance one or more wires of the second material composition.

As is indicated in Fig 2 as well as in Fig. 3, the body electrode 14 comprises a plurality of fluid transport passages 30 extending through both of the body-facing layer 22 and the conductive layer 24. The purpose of the fluid transport passages 30 is to guide exudate from the wound area (not shown in Fig. 2 or Fig.3) towards an absorbent article (not shown in Fig. 2 or Fig.3) when the body electrode 14 is in use.

In embodiments of the body electrode, such as the embodiment illustrated in Fig. 2 and Fig. 3, the conductive body 26 may be isolated from the environment ambient of the body electrode 14. To this end, the body electrode 14 may comprise a cover layer 32 covering the conductive body 26. As a non-limiting example, the cover layer 32 may comprise a lacquer or varnish. Instead of, or in addition to a lacquer or varnish, the cover layer 32 may comprise a tape (not shown).

Fig. 2 further illustrates that the body electrode 14 extends in a longitudinal direction X, a transversal direction Y and a vertical direction Z. The longitudinal direction X and the transversal direction Y together define a first plane extension in a first plane P, see Fig. 2.

The embodiment of the body electrode illustrated in Fig. 2 has a planar extension in a first plane extension, i.e. in the first plane P, and comprises, along the planar extension, a first body electrode portion 14' and a second body electrode portion 14". In the Fig. 2 embodiment, the conductive body 26 is located within the first body electrode portion 14'.

Moreover, as may be gleaned from Fig. 3 each one of the first body electrode portion 14' and the second body electrode portion 14" may comprise a portion of each one of the body-facing layer 22 and the conductive layer 24.

Purely by way of example, fluid transport passages 30 may be located in the second body electrode portion 14" only, i.e. the first body electrode portion 14' may be free from any fluid transport passage.

As regards the size of the fluid transport passages, as a non-limiting example, the total area, in the first plane P, of all the fluid transport may be within the range of 10-25% of the total area of the second body electrode portion 14".

As another non-limiting example, the cross-sectional area, in the first plane P, of each one of the fluid transport passages 30 may be within the range of 0.5 to 30 mm². In example embodiments of the body electrode, the cross-sectional area, in the first plane P, of each one of the fluid transport passages 30 may be within the range of 5 to 20 mm². In the embodiment of the body electrode 14 illustrated in Fig. 2 and 3, each one of the fluid transport passages 30 has a circular cross-section. However, it is envisaged that in other embodiments of the body electrode 14, one or more of the transport passages 30 may have another shape, such as a rectangular or oval shape.

Furthermore, in the embodiment of the body electrode 14 illustrated in Fig. 2 and 3, each one of the fluid transport passages 30 extends in a direction that is substantially perpendicular to the first plane P. However, it is envisaged that in other embodiments of the body electrode 14, one or more of the transport passages 30 may have an extension that forms an angle with the normal of the first plane P.

Fig. 4 illustrates another embodiment of the body electrode 14. As may be gleaned from Fig. 4, the conductive body 26 is located within the conductive layer 24.

In the Fig. 2 embodiment of the body electrode 14, the conductive body 26 forms a closed path in the planar extension of the first plane P.

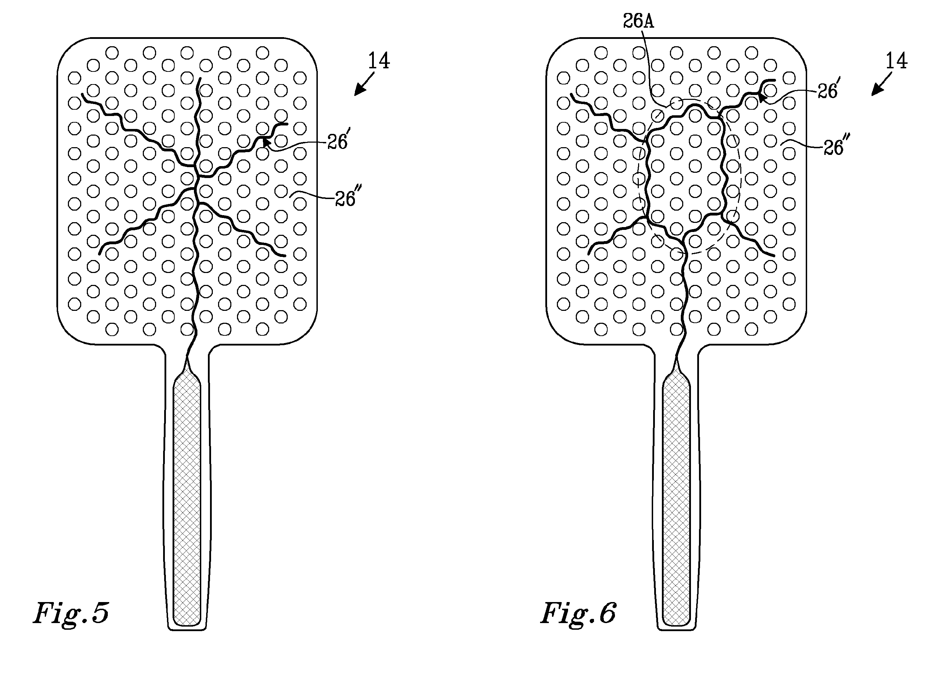

However, Fig. 5 illustrates an embodiment of the body electrode 14 in which the conductive body 26 comprises a plurality of conductive body branches 26', 26" that do not form part of a closed path.

Furthermore, Fig. 6 illustrates an embodiment of the body electrode 14 which comprises a conductive body 26 that comprises a first portion 26A that forms a closed path as well as a plurality of branches 26', 26" that do not form part of a closed path.

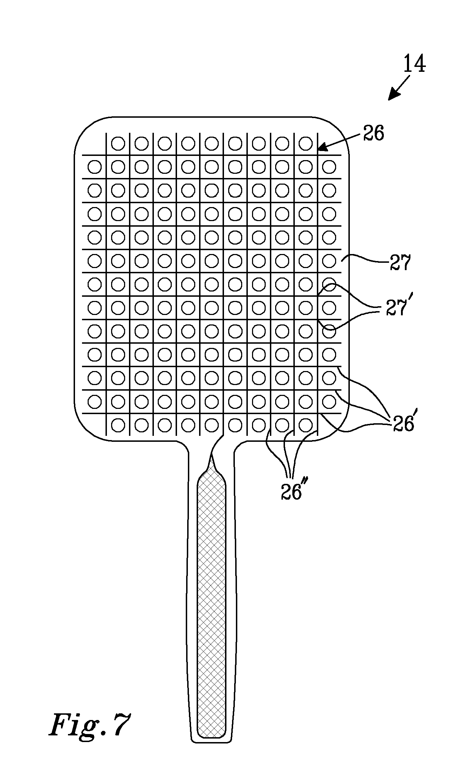

As another non-limiting example, Fig. 7 illustrates an embodiment of the body electrode 14 which comprises a conductive body 26 that comprises a mesh 27 or a web that comprises a plurality of branches 26', 26" that intersect one another at a plurality of nodes 27'.

Purely by way of example, a minimum distance d, along the planar extension of the first plane P, between the conductive body 26 and each one of the fluid transport passages 30 is within the range of 1 to 4 mm, preferably within the range of 2 to 3 mm. The minimum distance d is indicated in Fig. 2. As another non-limiting example, the minimum distance d may be equal to or greater than 1 mm, alternatively the minimum distance d may be equal to or greater than 2 mm.

The Fig. 2 embodiment of the body electrode 14 further comprises a lead 34 adapted to conduct an electrical current from a source of electrical current (not shown in Fig. 2) to the conductive body 26. Purely by way of example, the lead 34 may be elongate such that the length lₗ of the lead 34 is at least five times greater than the width l_{w} of the lead 34. Moreover, the width l_{w} of the lead 34 may be relatively small in relation to the width b_{w} of the remaining portion of the body electrode 14. The width l_{w} of the lead 34 extends in a first direction D₁ of the first plane P and the width b_{w} of the remaining portion of the body electrode 14 is the largest measure of the remaining portion of the body electrode 14 measured in the first direction D₁. As a non-limiting example, the width b_{w} of the remaining portion of the body electrode 14 may be at least three, alternatively at least five, times the width l_{w} of the lead 34. Purely by way of example, the width b_{w} of the remaining portion of the body electrode 14 may be within the range of 4 to 16 cm. Moreover, as a non-limiting example, the area of the body electrode 14, as measured in the first plane P, may be within the range of 40 to 200 cm².

In the Fig. 2 embodiment, the lead 34 comprises the second material composition. Moreover, in the Fig. 2 embodiment, the lead 34 and the conductive body 26 form a unitary component.

Moreover, though purely by way of example, the lead 34 may comprise an isolating layer covering at least a portion of the lead 34. Additionally, as a non-limiting example, the lead 34 comprise a layer of the first material composition.

As a non-limiting example, both the lead 34 and the conductive body 26 may be screen printed onto the conductive layer 24.

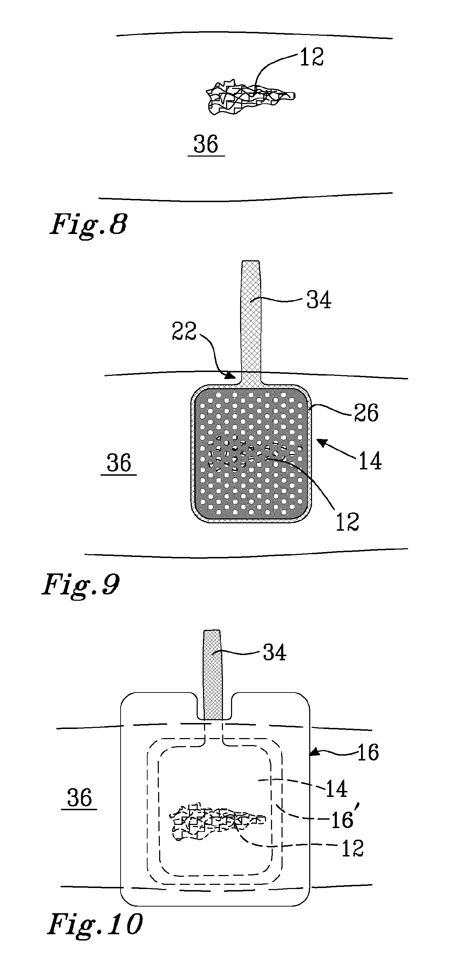

Fig. 8 to Fig. 14 illustrate various embodiments of a method of treating a wound using a body electrode 14. Purely by way of example, the body electrode 14 may be an embodiment of the body electrode 14 of the present invention.

Fig. 8 illustrates a wound area 36 comprising a wound 12. As a non-limiting example, prior to applying a body electrode 14 to the wound area 36, the wound 12 may firstly be cleansed. Moreover, though purely by way of example, the wound may also be filled with a wound filler such as a made of gauze, foam or hydrofiber.

Thereafter, and as is indicated in Fig. 9, a body electrode 14 is attached to the wound area 36 such that the body-facing layer 22 of the body electrode 14 faces the wound 12. Moreover, Fig. 9 illustrates that the embodiment of the body electrode 14 illustrated therein comprises a conductive body 26.

The body electrode 14 may optionally comprise a release liner (not shown) initially covering at least the body-facing layer 22 in order to ensure that the body-facing layer 22 is kept sterile prior to use. In such an event, the release liner is removed prior to applying the body electrode 14 to the wound 12.

Thereafter, the body electrode 14 is covered with an absorbent article 16, see Fig. 10, such that at least a portion of the absorbent article 16 faces a first side (not shown in Fig. 10) of the body electrode 14. The first side faces away from the body-facing layer 22.

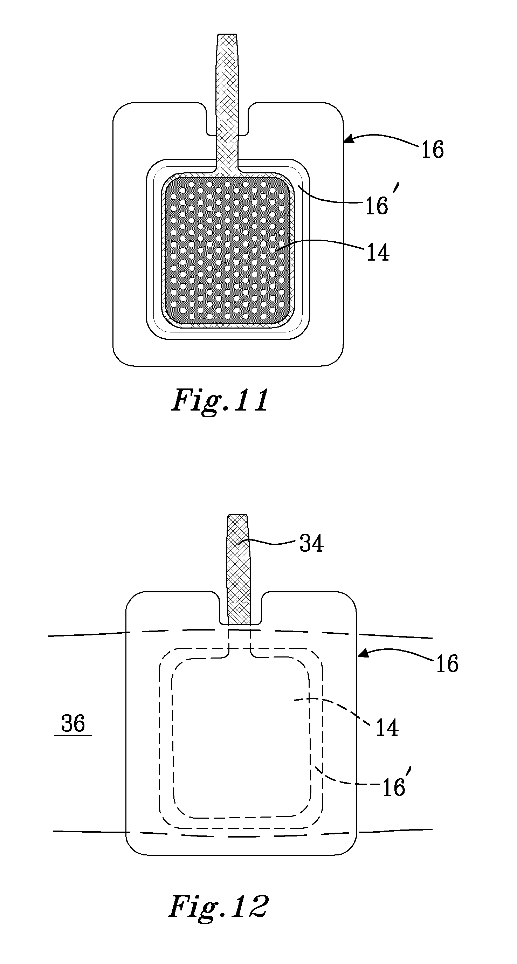

Fig. 9 and Fig. 10 illustrate an attachment sequence in which the body electrode 14 is applied to the wound 12 before the body electrode 14 is covered with the absorbent article 16. However, Fig. 11 and Fig. 12 illustrate an alternative attachment sequence in which the body electrode 14 is covered with the absorbent article 16 before the body electrode 14 is applied to the wound 12.

A portion of the body electrode 14, such as the lead 34 illustrated in each one Fig. 10 and Fig. 12 is preferably not covered by the absorbent article 16.

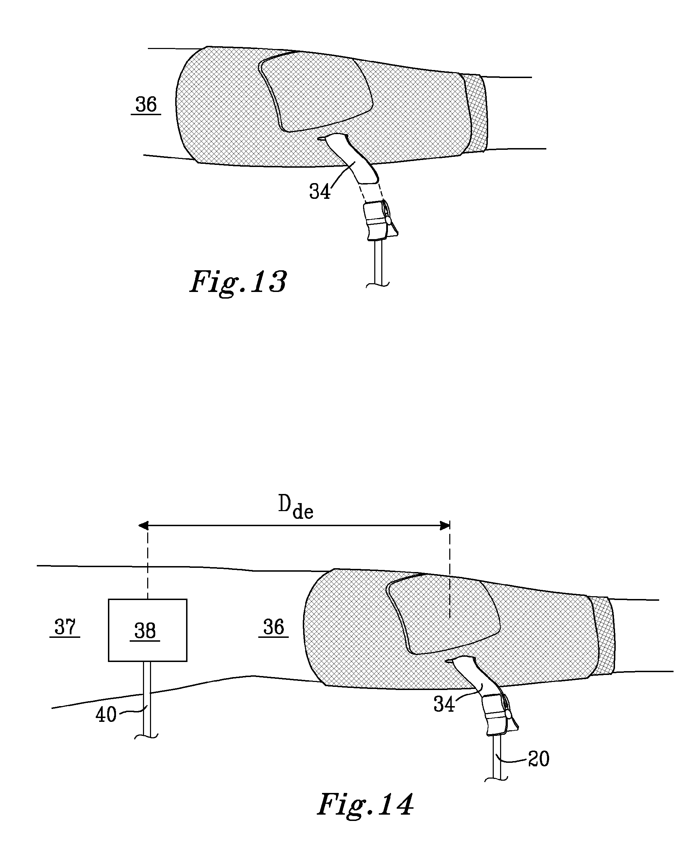

Once the body electrode 14 and the absorbent article 16 are connected to the wound area 36, they may, as a non-limiting example, be fixated by means of a fixator, see e.g. Fig. 13. Purely by way of example, the fixator may comprise a tubular or elastic bandage.

As an example, a portion of the body electrode 14, such as the lead 34, may be adapted to extend from the fixator such that that portion may be connected to a source of electric current, see Fig. 13.

Moreover, Fig. 14 illustrates that the method may comprise placing a disperser electrode 38 on the body 37 hosting the wound area to be treated at a disperser electrode distance D_{de} from the wound area. The disperser electrode distance D_{de} is greater than or equal to a predetermined disperser electrode distance value. As is intimated in Fig. 14, the disperser electrode distance D_{de} is measured from the centre of the disperser electrode 38 to the centre of the wound area. Purely by way of example, the predetermined disperser electrode distance value may be within the range of 20 to 40 cm. Moreover, the method may comprise that an electrical current is applied to the disperser electrode 38, for instance via a disperser cable assembly 40 comprising on or more cables.

The disperser cable assembly 40 and the body electrode cable 20 may be connected to the same source of electrical current such that the disperser electrode 38 and the body electrode 14 form a closed circuit via a portion of the body 37 hosting the wound area.

Finally, it should be recognized that structures and/or elements and/or method steps shown and/or described in connection with any disclosed form or embodiment of the invention may be incorporated in any other disclosed or described or suggested form or embodiment as a general matter of design choice.

## Claims

1. A body electrode (14) comprising a body-facing layer (22), a conductive layer (24) and a conductive body (26), said conductive layer (24) comprising a first material composition and said conductive body (26) comprising a second material composition, said second material composition having a lower electrical resistance than said first material composition, said conductive body (26) being located within and/or on said conductive layer (24), said body electrode (14) comprising a plurality of fluid transport passages (30) extending through both of said body-facing layer (22) and said conductive layer (24).

2. The body electrode (14) according to claim 1, wherein said conductive body (26) is attached to a first side of said conductive layer (24), said first side facing away from said body-facing layer (22).

3. The body electrode (14) according to claim 1 or 2, wherein said conductive body (26) is isolated from the environment ambient of said body electrode (14).

4. The body electrode (14) according to any one of claims 1 to 3, wherein said body electrode (14) has a planar extension in a first plane extension and comprises, along said planar extension, a first body electrode portion (14') and a second body electrode portion (14"), said conductive body (26) being located within said first body electrode portion (14').

5. The body electrode (14) according to claim 4, wherein each one of said first and second body electrode portions (14', 14") comprises a portion of each one of said body-facing layer (22) and said conductive layer (24)

6. The body electrode (14) according to any one of the preceding claims, wherein said body-facing layer (22) comprises a hydrogel.

7. The body electrode (14) according to claim 6, wherein said hydrogel has a liquid content within the range of 25% to 50%.

8. The body electrode (14) according to any one of the preceding claims, wherein said body-facing layer (22) has a thickness within the range of 0.1 to 1.0 mm.

9. The body electrode (14) according to any one of the preceding claims, wherein the cross-sectional area of each one of said fluid transport passages (30) is within the range of 0.5 to 30 mm².

10. The body electrode (14) according to any one of the preceding claims, wherein said first material composition comprises an intrinsic conductive carbon material.

11. The body electrode (14) according to any one of the preceding claims, wherein said second material composition comprises a metal or an alloy.

12. The body electrode (14) according to claim 11, wherein said second material composition is constituted by a metal or an alloy.

13. The body electrode (14) according to any one of claims 2 to 12, wherein said conductive body (26) is attached to said first side of said conductive layer (24) by means of screen printing and/or roll coating.

14. The body electrode (14) according to any one of claims 3 to 13, wherein said conductive body (26) is isolated from the environment ambient of said body electrode (14) by means of an isolating layer covering said conductive body (26).

15. The body electrode (14) according to any one of claims 4 to 14, wherein said conductive body (26), in said planar extension, forms a closed path.

16. The body electrode (14) according to any one of claims 4 to 15, wherein a minimum distance, along said planar extension, between said conductive body (26) and each one of said fluid transport passages (30) is within the range of 1 to 4 mm, preferably within the range of 2 to 3 mm.

17. The body electrode (14) according to any one of the preceding claims, wherein said body electrode (14) further comprises a lead adapted to conduct an electrical current from a source of electrical current to said conductive body (26).

18. The body electrode (14) according to claim 17, wherein said lead comprises said second material composition.

19. A wound care treatment kit, said kit comprising a body electrode (14) according to any one of the preceding claims, said kit further comprising an absorbent article (16) adapted to be attached to said body electrode (14), at least a portion of said absorbent article being adapted to face a first side of said conductive layer (24), said first side facing away from said body-facing layer (22).

20. A method for treating a wound (12) in a wound area (36), said method comprising:
- applying a body electrode (14) according to any one of claims 1 to 18 to said wound area (36) such that said body-facing layer (22) faces said wound (12);
- covering said body electrode (14) with an absorbent article (16) such that at least a portion of said absorbent article faces a first side of said conductive layer (24), said first side facing away from said body-facing layer (22), and
- applying an electrical current to said conductive body (26).

21. The method according to claim 20, wherein said body electrode (14) is applied to said wound area (36) before said body electrode (14) is covered with said absorbent article (16).

22. The method according to claim 20, wherein said body electrode (14) is covered with said absorbent article (16) before said body electrode (14) is applied to said wound area (36).

23. The method according to any one of claims 20 to 22, wherein said body electrode (14) comprises a release liner initially covering at least said body-facing layer (22), said method comprising removing said release liner prior to applying said body electrode (14) to said wound area (36).

24. The method according to any one of claims 20 to 23, wherein said method comprises fixating said body electrode (14) and said absorbent article by means of a fixator.

25. The method according to any one of claims 20 to 24, when dependent on either one of claims 17 or 18, wherein said method comprises connecting said lead to a source of electric current.

26. The method according to any one of claims 20 to 25, wherein said method further comprises:
- placing a disperser electrode (38) on the body 37 hosting said wound area at a disperser electrode distance (D_{de}) from said wound area, said disperser electrode distance (D_{de}) being greater than or equal to a predetermined disperser electrode distance value, and
- applying an electrical current to said disperser electrode (38).
